(19) Europäisches Patentamt
European Patent Office
Office européen des brevets

(11) Publication number: **0 148 616 B1**

(12) # EUROPEAN PATENT SPECIFICATION

(45) Date of publication of patent specification: **17.04.91**

(51) Int. Cl.[5]: **C07F 1/02**, C07J 1/00, //C07J5/00,C07J7/00

(21) Application number: **84308826.1**

(22) Date of filing: **17.12.84**

Divisional application 90115678.6 filed on 17/12/84.

(54) **Stabilised monolithium acetylide and the ethynylation of steroids.**

(30) Priority: **05.01.84 US 568558**
**20.03.84 US 591631**
**20.04.84 US 602300**
**26.04.84 US 604091**

(43) Date of publication of application:
**17.07.85 Bulletin 85/29**

(45) Publication of the grant of the patent:
**17.04.91 Bulletin 91/16**

(84) Designated Contracting States:
**BE CH DE FR GB IT LI NL SE**

(56) References cited:
**EP-A- 0 019 247**
**EP-A- 0 104 054**

**JOURNAL OF ORGANIC CHEMISTRY, vol. 43, no. 24, 24th November 1978, pages 4679-4680, American Chemical Society, Washington DC, US; G. NEEF et al.: "Revision of some 16-alkylated steroids"**

(73) Proprietor: **THE UPJOHN COMPANY**
**301 Henrietta Street**
**Kalamazoo, Michigan 49001(US)**

(72) Inventor: **Timko, Joseph Michael**
**c/o The Upjohn Company 301 Henrietta Street**
**Kalamazoo Michigan 49001(US)**
Inventor: **van Rheenen, Verlan Henry**
**c/o The Upjohn Company 301 Henrietta Street**
**Kalamazoo Michigan 49001(US)**
Inventor: **Cha, Dae Yang**
**c/o The Upjohn Company 301 Henrietta Street**
**Kalamazoo Michigan 49001(US)**

(74) Representative: **Perry, Robert Edward et al**
**GILL JENNINGS & EVERY 53-64 Chancery Lane**
**London WC2A 1HN(GB)**

**EP 0 148 616 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

JOURNAL OF MEDICINAL CHEMISTRY, vol. 11, no. 5, August 1968, pages 924-928, Washington DC, US; D.K. PHILLIPS et al.: "Estra-1,3,5(10),15-tetraenes. I. Birch reduction"

JOURNAL OF ORGANIC CHEMISTRY, vol. 40, no. 15, 25th July 1975, pages 2250-2252, Washington DC, US; M.M. MIDLAND: "Preparation of monolithium acetylide in tetrahydrofuran. Reaction with aldehydes and ketones"

## Description

This invention relates to stabilised monolithium acetylide and the ethynylation of steroids.

The ethynylation of 17-keto steroids, to give commercially-important 17α-ethynyl-17β-hydroxy steroids, is well known and described in, for example, US-A-2272131, US-A-2843609, US-A-2723280, US-A-2877240, US-A-3275666, US-A-3470217, US-A-4041055, "Steroids", by Fieser and Fieser, Reinhold Publishing Co., New York (1959) 557-591, and J.A.C.S. 78 (1956) 2477.

One known method of ethynylation comprises reacting the 17-keto steroid with dipotassium acetylide. An advantage of this method is that $\Delta^4$-3-keto steroids can be ethynylated, without there being any need to protect the 3-keto group. However, it is well known that this method cannot be used to ethynylate 16α-methyl-17-keto, 16β-methyl-17-keto or 16-methylene-17-keto steroids; these are reactions of importance because the corresponding 17α-ethynyl steroids can be transformed to dexamethasone, betamethasone and melengestrol acetate.

Metal acetylides other than dipotassium acetylide are known. Monosodium acetylide is disclosed in US-A-3470217 and by Tedeschi et al., J. Org. Chem. 34 (1969) 435. Mono- and bis-magnesium acetylides are disclosed by Skattebol et al., J.A.C.S. (1956) 4765; US-A-3704253 discloses their use for 17α-ethynyl introduction, but there is substantial dimer formation.

In many cases, lithioacetylide reagents exhibit substantially different reactivity from other metal acetylides. This fact, and the ready availability of n-butyllithium, have resulted in the extensive use of these reagents in syntheses. The covalent nature of the carbon-lithium bond has been the subject of many theoretical and experimental investigations; see, for example, Moffat, J. Mol. Structure 42 (1977) 251, and Streitwieser et al ., J.A.C.S. 98 (1976) 4778.

Midland, J. Org. Chem. 40 (1975) 2250, reacted n-butyllithium with acetylene in THF at low temperature (<-70°) and in dilute solution to produce monolithium acetylide. Monolithium acetylide is a valuable reagent for the preparation of ethynyl carbinols and terminal acetylenes, see Fieser and Fieser, Reagents for Organic Synthesis, Vol. 1, Wiley, New York, 1967, p 573. Midland found that warming or attempting to generate a more concentrated solution resulted in disproportionation to the insoluble dilithium acetylide and acetylene. This disproportionation is an important disadvantage and occurs in the absence of a complexing agent, see Corbellini et al., Chem. Ind. (Milan) 42, 251 (1960) and Chem Abstr. 54, 19250 (1960). To reduce or prevent the disproportionation the monolithium acetylide is usually prepared in liquid ammonia, which presumably serves as an appropriate complexing agent. An amine such as ethylenediamine can also be used to stabilize monolithium acetylide. Ethylenediamine so greatly stabilizes monolithium acetylide that monolithium acetylide is sold commercially as an ethylenediamine complex. Ethylenediamine while stabilizing monolithium acetylide to the point it can be sold commercially actually reduces the reactivity to the point it is not useful for many ethynylation procedures.

The addition of a lithiated acetylene species to a 16β-methyl-17-ketone in 92% yield was reported by G. Neef, et al., in J. Org. Chem. 43, 4679 (1978) without giving any experimental data but stating, "The ethynylations were performed according to the procedure of Phillips..." The procedure of Phillips is set forth in J. Med. Chem. 11, 924 (1968). The results reported by Neef could not be reproduced; the Phillips procedure consistently gave large amounts (greater than 20%) of irreversible enolization. Neef also reported the addition of a lithiated acetylene species to a 16α-methyl-17-keto steroid. The yield Neef reported (72%) is more in keeping with the observed irreversible enolization.

US Patent 4,055,562 used monolithium acetylide to ethynylate 17-keto steroids unsubstituted in the $C_{16}$ position. The monolithium acetylide was prepared by bubbling acetylene into THF held at -70° under anhydrous conditions followed by addition of butyllithium. The 17-keto steroid was added to the unstabilized monolithium acetylide and the mixture stirred for 3 hr at -70° to produce the 17α-ethynyl-17β-hydroxy steroid product.

US-A-4320236 discloses the use of a monolithium acetylide-ammonia complex to ethynylate unsaturated acyclic ketones at below 30°C; temperatures of -50 to 10°C are reported in the Examples. The unsaturated acyclic ketones are very reactive; 16-methyl-17-keto steroids, being highly-substituted sterically-hindered cyclopentanones, are much less reactive.

It would be desirable to provide a stabilised monolithium acetylide which can be prepared, and react, at up to 0°C, with 16α- and 16β-methyl- and 16-methylene-17-keto steroids to produce 16-substituted-17α-ethynyl-17β-hydroxy steroids, preferably in high yield and with little or no epimerisation or destruction of the 16-methylene group.

EP-A-0104054 is in the state of the art according to Article 54(3) EPC. It discloses the reaction of a $C_3$-protected 16-methylene-17-keto steroid with an excess of monolithium acetylide cooled to -20°C or below. In particular, Examples 33 and 36 disclose triethylamine-and diisopropylamine-stabilised monolithium

acetylide, reacted with 3-methoxy-16-methyleneandrosta-3,5-dien-17-one, respectively in situ and after preparing the stabilised acetylide.

According to a first aspect of the present invention, in anhydrous solution of an amine-stabilised monolithium acetylide, the amine is selected from N,N-diisopropylethylamine, t-butylamine, diethylamine, dicyclohexylamine, hexamethyldisilazane and pyrrolidine.

Such a solution can be prepared by a (two-pot) process which comprises contacting the amine with an organolithium compound at 0° C or below, and contacting the resultant product with a solution of acetylene in an anhydrous solvent at 0° C or below.

According to another aspect of the invention, a (one-pot) process for preparing an anhydrous solution of an amine-stabilised monolithium acetylide, wherein the amine is selected from N,N-diisopropylethylamine, triethylamine, diisopropylamine, t-butylamine, diethylamine, dicyclohexylamine, hexamethyldisilazane and pyrrolidine, comprises contacting the amine with a solution of acetylene in an anhydrous solvent, and contacting the resultant product with an organolithium compound at 0° C or below.

The invention also provides processes for preparing a steroid of formula IIA, IIB, IIC, IVA, IVB or IVC, $C_3$-protected if necessary (see formula charts A and B), either by (1) contacting a corresponding steroid of formula IA, IB, IC, IIIA, IIIB or IIIC, respectively, with a solution of acetylene in an anhydrous solvent, (2) contacting an amine as defined above with an organolithium compound at 0° C or below, and (3) contacting the respective mixtures at 0° C or below; or (1) contacting the corresponding steroid with monolithium acetylide stabilised by an amine as defined above, at -10° C or below, and (2) quenching the reaction while maintaining the temperature at -10° C or below. If the starting material is of formula IIIA, it is not 3-methoxy-16-methyleneandrosta-3,5-dien-17-one.

The 16-methylene-17-keto steroid (III) starting materials are well known to those skilled in the art or can be readily prepared from known compounds by methods well known to those skilled in the art. For example, 16-methylene-17-keto steroids (III) where $R_6$ is methyl, α-fluoro and methylene are known, see U.S. Patents 3,166,551, 2,838,942 and 3,980,778 respectively. 16-Methylene-17-Keto steroids (I) with C-ring substitution are known, for example, 9α-hydroxy (U.S. Patent 3,065,146), $\Delta^{9(11)}$ (U.S. Patent 4,127,596), 11β-hydroxy and 11-keto (U.S. Patent 2,867,630). Further, these steroids are known in the $C_3$ protected forms, for example, the enol ether (U.S. Patent 3,516,991) and the 3-enamine (U.S. Patent 4,216,159).

The 16-methylene-17-keto steroid (III) starting materials are well known to those skilled in the art. See, for example, U.S. Patents 3,275,666, 3,300,521 and 3,641,069, Gazz. Chim. Ital . 91, 672 (1961), Hungarian Patent 019,495 and U.S. Patent 4,416,821.

It is preferred that the 16-methylene-17-keto (III) starting materials be prepared by the process of US Patent 4,416,821.

The 16-methylene-17-keto steroids (IIIA-C) starting materials may have variable substituents at positions 1, 6, 9, 10 and 11, as is well known to those skilled in the art. For example, U.S. Patent 4,416,821 discloses 16-methylene-17-keto steroids with $\Delta^1$, 6-fluoro, 6-methyl, 11β-hydroxy, 11-keto, 11α-hydroxy, $\Delta^{9(11)}$, 9β,11β-epoxy, and 9α-fluoro substitution as well as combinations thereof. It is preferred in the $\Delta^4$-3-keto series (A) that $R_6$ be a hydrogen atom, methyl or methylene group but in the $\Delta^{1,4}$-3-keto series (B) that $R_6$ be a hydrogen or fluorine atom.

The 16α- and 16β-methyl-17-keto steroids (I) are well known to those skilled in the art or can be readily prepared by methods well known to those skilled in the art from known compounds. See, for example, U.S. Patents 3,010,958, 3.039,528 and 3,704,253. For the 16β-methyl-17-keto steroids (I) P. deRuggieri, et al ., Gazz. Chim. Ital . 91, 672 (1961) on page 678 disclosed a 3-step process for the transformation of a 17-keto steroid to the corresponding 16β-methyl-17-keto steroid (I) in about 40% yield. The yield of that reaction can be greatly increased, and it is preferred to prepare the 16β-methyl-17-keto steroids from 17-keto steroids by the process of US Patent 4,451,404.

The 17-keto steroids (I) starting materials are well known to those skilled in the art or can be readily prepared from known compounds by methods well known to those skilled in the art. For example, 17-keto steroids (I) where $R_6$ is methyl, α-fluoro and methylene are known, see U.S. Patents 3,166,551, 2,838,942 and 3,980,778 respectively. 17-Keto steroids (I) with C-ring substitution are known, for example, 9α-hydroxy (U.S. Patent 3,065,146), $\Delta^{9(11)}$ (U.S. Patent 4,127,596), 11β-hydroxy and 11-keto (U.S. Patent 2,867,630). Further, these steroids are known in the $C_3$ protected forms, for example, the enol ether (U.S. Patent 3,516,991) and the 3-enamine (U.S. Patent 4,216,159).

The 17-keto steroids (IA-IC) may or may not have to have the functionality at $C_3$ protected during the ethynylation reaction depending on the nature of the steroid A ring (A-C, see Charts B and C). For the $\Delta^4$-3-keto steroids (A), the $C_3$ ketone is protected as the enol ether (Aa), ketal (Ab), or enamine (Ac) as is well known in the art, see Chart C. The preferred enol ether (Aa) is the methyl or ethyl ether. The preferred ketal (Ab) is the ethylene ketal. The preferred enamines (Ac) are selected from the group consisting of

pyrrolidine, morpholine and diethylamino amines. The enol ethers (a) are prepared by methods well known in the art, see J. Org. Chem. 26, 3925 (1961), Steroid Reactions, Edited by Carl Djerassi, Holden-Day, San Francisco 1963, page 42-45, and U.S. Patent 3,516,991 (Preparation 1). The ketals (b) are also prepared by well known methods, see Steroid Reactions, supra, page 11-14. The 3-enamines (c) are also prepared by methods well known in the art, see U.S. Patents 3,629,298 and 4,216,159 and Steroid Reactions, supra, page 49-53.

The $\Delta^{1,4}$-3-keto steroids (B) do not have to have the $C_3$ ketone protected.

The 3-hydroxy steroid (C) should have the 3β-hydroxyl group protected as the ether (Ca), see Chart C. The preferred blocking groups are the methyl, ethyl, tetrahydropyranyl (THP) and trimethylsilyl ethers.

Monolithium acetylide is known, see M. M. Midland, J. Org. Chem. 40, 2250 (1975) and US Patents 4,055,562 and 4,320,236.

The monolithium acetylide can be prepared in different ways which is of importance because the temperature used in preparation is dependent on the method of preparation.

The monolithium acetylide can be prepared by bubbling acetylene through an etheral solvent such as THF at about 20-25° until no further weight gain occurs (about 0.6M in acetylene). An aliquot of this solution is cooled to about -60° and 4 equivalents (per equivalent of steroid) of an organo-lithium reagent such as n-butyllithium, methyllithium or phenyllithium are added with vigorous stirring to give a mixture which is about 0.57M in monolithium acetylide. Temperatures above about -10° and concentrations above about 0.8M in monolithium acetylide cause disproportionation to the insoluble dilithium acetylide. Therefore, slow addition of pre-cooled (-20° or less) organo-lithium reagent to very cold solution of acetylene in a dry solvent such as THF, diethyl ether, dioxane, and DME is preferred. It is preferred the temperature be kept at -80 to -25°, more preferably at -80 to -40°. If the organic solvent is at a temperature of -40° or warmer, the organo-lithium solution should be added slower using a higher stirring rate to facilitate heat transfer. The monolithium acetylide solution should be used immediately after preparing as letting it stand even at -78° for 6 hours may lower the yield 10%.

A stabilized monolithium acetylide as used in the instant case permits preparation of the monolithium acetylide at higher temperatures which is commercially advantageous. The stabilized monolithium acetylide is prepared in 3 alternative ways, two-pot (preferred), one-pot or in situ.

In the two-pot process, a sufficient quantity of acetylene is first dissolved in a suitable dry organic solvent. The temperature at which the acetylene can be dissolved in the dry organic solvent is not critical. The temperature affects the solubility and therefore the concentration of the acetylene. However, before the acetylene solution is contacted with the lithium complex it must be cooled to 0° or less. The nature of the organic solvent is not critical so long as it does not react with acetylene, organolithium compounds or amines. Suitable dry organic solvents include THF, dioxane, diethyl ether, t-butyl methyl ether and dimethoxyethane. The preferred solvent is THF. It is convenient for the organic solvent to be saturated with acetylene. Second, an organo-lithium compound is contacted with a stabilizing amine at 0° or less in a suitable dry organic solvent. Organo-lithium compounds include n-butyllithium, phenyllithium, and methyllithium, preferred is n-butyllithium. A stabilizing amine is an amine (primary, secondary or tertiary) which when reacted with an organo-lithium compound to form a lithium complex and/or corresponding lithium amide subsequently reacted with acetylene forms a stabilized monolithium acetylide which will not significantly disproportionate at 0° and below which is reactive towards 17-keto steroids. Stabilizing amines are N,N-diisopropyl ethyl amine, triethylamine, diisopropyl amine, t-butyl amine, diethylamine, dicyclohexylamine, hexamethyl disilazane, and pyrrolidine. Some amines such as pyrrolidine are only useful with some steroids such as 16β-methylandrosta-1,4,9(11)-triene-3,17-dione. Amines which are not operable include ethylenediamine and pyridine. Preferred is diisopropylamine or triethylamine. The same dry organic solvents useful to dissolve the acetylene in are also useful here. It is preferred that the same solvent or mixture of solvents be used for dissolving the acetylene in as for the reaction of the organo-lithium compound with the stabilizing amine. The reaction of the organo-lithium compound with a primary or secondary amine produces a lithium amide. A tertiary amine cannot produce a lithium amide and forms a complex, the nature of which is not known. Therefore, the reaction of an organo-lithium compound and a stabilizing amine will be considered to have produced a lithium complex. When the organo-lithium compound is added to the stabilizing amine it must be added slowly maintaining the temperature at about 25° or less. The reaction is complete in less than 30 min.

The third step of the two-pot process is contacting the lithium complex of step 2 with the acetylene solution of step 1 at 0° or less. It is preferred that the method of contacting be a slow addition of the lithium complex to the acetylene solution keeping the temperature at 0° or less. The reaction forming monolithium acetylide is complete in about 30 min.

In the two-pot process, the first two steps which are independent of each other can be performed in the

reverse order. The organolithium compound can be reacted with the stabilizing amine prior to the dissolving of the acetylene in the dry organic solvent. All that is necessary is that both steps be independently performed and the temperature of both mixtures be 0° or less before they are combined in the third step.

The stabilized monolithium acetylide produced by the process of the present invention is reactive with aldehydes and ketones. It is preferred that the reactant be a 17-keto steroid. Commercially one of the most important uses of the ethynylation process is the transformation of 17-keto steroids to the corresponding 17α-ethynyl-17β-hydroxy steroid. For example, androstenedione, a 17-keto steroid (protected as the 3-enol ether) is ethynylated to ethisterone (Example 1). Ethisterone by the process of US Patent 4,041,055 (Examples 1, 5, 9 and 13) is converted to 17α-hydroxyprogesterone. Androstenedione is a $\Delta^4$-3-keto steroid and can be ethynylated with dipotassium acetylide (preferably) or monolithium acetylide. In Preparation 1 of US Patent 4,041,055 monolithium acetylide ethylene diamine was used, it is sufficiently reactive to react with androstenedione which has no substitution at $C_{16}$ but is not sufficiently reactive to react with 16-substituted-17-keto steroids such as 16α-methyl-, 16β-methyl- or 16-methylene-17-keto steroids without serious side reactions.

The monolithium acetylide produced by the process of the present invention is sufficiently stable at up to about 0° and is reactive with 16α-methyl-, 16β-methyl and 16-methylene-17-keto steroids. 16α-methyl- and 16β-methyl-17-keto steroids are well known to those skilled in the art. 16-Methylene-17-keto steroids are also known, see for example US Patent 4,416,821.

The 16-methyl-17-keto or 16-methylene steroids (I or III) to be ethynylated are added to the monolithium acetylide at 0° or less. The ethynylation reaction is complete in about 30 minutes. The 16-methyl-17-keto (I) or 16-methylene-17-keto steroid (III) can be added as a solid, a slurry or as a solution. For convenience it is preferred that it be added as a solution. It should be added slowly as is known to those skilled in the art. Again, the same organic solvents used for steps 1 and 2 are useful for adding the material to be ethynylated to the monolithium acetylide. Again, it is preferable to use the same organic solvent as was used in steps 1 and 2. The ethynylated product is recovered by means well known to those skilled in the art.

In the one-pot process, the first step of dissolving the acetylene in a suitable dry organic solvent is the same as for the two-pot process. Second, the stabilizing amine is added to the acetylene solution at 0° or less. Third, the organo-lithium compound is added slowly to the mixture of the acetylene solution and the stabilizing amine again at 0° or less.

With the in situ process, the first step again is the same. Second, the 16-methyl-17-keto (I) or 16-methylene steroid (III) to be ethynylated is added to the acetylene solution at 0° or less. Third, the organolithium compound and the stabilizing amine are reacted to form the lithium complex as in the second step of the two-pot process. Finally, the lithium complex is added slowly to the mixture of the acetylene solution containing the 16-methyl-17-keto (I) or 16-methylene steroid (III) to be ethynylated at 0° or less and the monolithium acetylide is generated in situ.

While the above steps to prepare monolithium acetylide can be performed at 0° it is preferable to perform them at less than -20°, more preferable in a temperature range of about -20° to about -40°. Reducing the temperature reduces the amount of disproportionation, thereby increasing the amount of monolithium acetylide available for ethynylation.

Regardless of how the monolithium acetylide is prepared (two-pot, one-pot or in situ) when it is contacted with the $C_3$ protected 17-keto-16-methyl (I A,C) or 16-methylene steroids (III A,C) at about 0° or less or the unprotected $\Delta^{1,4}$-3-keto 17-keto-16-methyl (IB) or 17-keto-16-methylene (IIIB) steroids at about -20° or less, the reaction time is very short, in the range of 5-60 min usually about 10-15 min.

The above discussion involves the temperatures at which the monolithium acetylide is prepared. The main advantage of using stabilized monolithium acetylide over non-stabilized monolithium acetylide is that it can be prepared and reacted at warmer temperatures. The reaction temperature of the monolithium acetylide with the 16α- and 16β-methyl-17-keto (I) or 16-methylene-17-keto steroids (III) is usually about the same as the preparation temperature. If the reaction is performed above the decomposition temperature of the reagent, the yield will decrease. In the $\Delta^{1,4}$-3-keto series (B) the reaction temperature must be lower than in the protected $\Delta^4$-3-keto (A) or 3-hydroxy (C) series because one must have selectivity for the monolithium acetylide with the $C_{17}$-ketone over the unprotected $C_3$-ketone. With the 16-methyl $\Delta^4$-3-keto (IA) and 3-hydroxy (IC) steroids in their $C_3$-protected form the ethynylation reaction is performed at about -10° or less, preferably at about -20° or less, more preferably at about -40° or less. With the 16-methyl-$\Delta^{1,4}$-3-keto steroids (IB) the ethynylation reaction is performed at about -10° or less, preferably at about -40° or less, more preferably at about -70° or less. With the 16-methylene-$\Delta^4$-3-keto (IIIA) and 3-hydroxy (IIIC) steroids in their $C_3$-protected form the ethynylation reaction is performed at about 0° or less, preferably at about -20° or less, more preferably at about -40° or less. With the 16-methylene-$\Delta^{1,4}$-3-keto

steroids (IIIB) the ethynylation reaction is performed at about -20° or less, preferably at about -40° or less, more preferably at about -50° or less.

With the $\Delta^{1,4}$-3-keto-16-methyl-17-keto (IB) and $\Delta^{1,4}$-3-keto-16-methylene steroids (111B) no $C_3$ protecting group is necessary. However, it is preferable to add at least one equivalent of lithium ion prior to contacting the 17-keto steroid (I) with the monolithium acetylide. The lithium ion helps keep the selectivity for the 17-ketone over the 3-ketone. The lithium ion is added as a lithium salt, for example, lithium chloride, lithium sulfate, etc. Preferred is lithium bromide or lithium perchlorate. Using the lithium ion with $\Delta^{1,4}$-3-keto-16-methyl-17-keto (IB) and $\Delta^{1,4}$-3-keto-16-methylene steroids (IIIB) steroids permits the same yield and selectivity at -40° as was obtained without the lithium ion at -78°.

Generally, slightly more than one equivalent of monolithium acetylide per equivalent of 16-methyl-17-keto (I) or 16-methylene-17-keto steroid (III) is used. With the 16α-methyl-, 16β-methyl-and 16-methylene-17-keto steroids (I and III) the more monolithium acetylide used up to 4 equivalents the better. Two equivalents are preferred and about four equivalents are more preferred.

When the acetylene addition is complete the excess acetylide is quenched or destroyed by reaction with a quenching agent which is any system such as water, saline or aqueous buffers depending on what final pH is desired. The quenching agent can be any system which protonates the acetylide, e.g. water, saline, buffers or non-aqueous systems containing protonating compounds, e.g. acetic acid, formic acid or other acid. Other quenching agents which are operable are those which will react with the acetylide such as acetone, methyl ethyl ketone or other ketones. The preferred quenching agent is acetone followed by aqueous acid. The 17α-ethynyl-17β-hydroxy-16-methyl (II) and 17α-ethynyl-17β-hydroxy-16-methylene steroids (IV) are obtained or isolated from the reaction mixture by means well known to those skilled in the art. In the case of the $\Delta^4$-3-keto steroids (A) and 3-hydroxy steroid (C) the 17α-ethynyl-17β-hydroxy steroids (IIA, IIC, IVA and IVC) are isolated as the $C_3$ protected form. The $C_3$ protecting group is removed by means well known to those skilled in the art or the $C_3$ protecting group may be left on for further chemical modification. Before the 17α-ethynyl-17β-hydroxy steroid (II or IV) is isolated the $C_3$ protecting group can be hydrolyzed in situ so as to obtain the unprotected or free 17α-ethynyl-17β-hydroxy steroid by reaction with a proton source such as sulfuric acid or hydrochloric acid at 20-25°. The acid is added until the pH is less than 2. For example, if the steroid (IA or IIIA) is protected as the enol ether (a) the protecting group can be removed by acid so that the 17α-ethynyl-17β-hydroxy steroid (II or IV) will be isolated in the free $\Delta^4$-3-keto form (A). The $\Delta^{1,4}$-3-keto steroids (B) are not protected and therefore the 17α-ethynyl-17β-hydroxy product (II or IV) will be in the free or unprotected form. The $C_3$ protecting group is removed from the 3-hydroxy steroids (C) by reaction with a means for hydrolyzing the $C_3$ protecting group which in the case of the ethers (Ca) includes acids with a pKa of less than 4.0.

The reaction mixture is worked up by adding saline to the hydrolysis mixture which results in 2 phases. The organic phase is separated, washed, dried and concentrated to give the desired product. Alternatively the product may be knocked out by water addition and recovered by known procedures.

The 17α-ethynyl-17β-hydroxy-16-methyl steroids (II) are useful as intermediates in the preparation of 16-methyl-17α-hydroxyprogesterones and 16-ethyl corticoids, see U.S. Patent 4,041,055. For example, 17α-ethynyl-17β-hydroxy-16β-methylandrosta-1,4,9(11)trien-3-one (Examples 2 and 14) is transformed to 17α,21-dihydroxy-16β-methylpregna-1,4,9(11)-triene-3,20-dione by the process of U.S. Patent 4,041,055. This $\Delta^{9(11)}$-corticoid is transformed by known methods to the bromohydrin, 9β,11β-epoxide and finally to the corresponding 9α-fluoro-11β-hydroxy compound which is betamethasone (US Patent 3,485,854).

The 17α-ethynyl-17β-hydroxy-16-methylene steroids (IV) are useful intermediates in the preparation of 17α-hydroxy-16-methylene-progesterones, see Chart D. The 17α-ethynyl-17β-hydroxy-16-methylene steroid (IV) is transformed to the corresponding 17β-hydroxy steroid (V) by reaction with a mercuric agent. Oxymercuration of ethisterone derivatives is old, see Helv. Chim. Acta. 26, 680 (1943). However, the present D ring is not a simple ethisterone derivative. Here the 17α-ethynyl-17β-hydroxy substituents are allylic to a 16-methylene group. Surprisingly and unexpectedly quantitative yields of the 17β-hydroxy steroids (V) are obtained, indicating that its allylic alcohol system did not compete with the propargyl alcohol system in the oxymercuration.

The definitions and explanations below are for the terms as used throughout the specification.

All temperatures are in degrees Centigrade.

TLC refers to thin-layer chromatography.

THF refers to tetrahydrofuran.

Monolithium acetylide refers to $LiC_2H$ and complexed forms thereof.

$R_3$ is $C_{1-5}$ alkyl, with the proviso that for the ketal (Ab) and the enamine (Ac), the $R_3$ groups can be the same or different and can be connected.

$R_3'$ is $C_{1-3}$ alkyl, $-Si(CH_3)_3$, tetrahydropyranyl or $-OCH_2CH_2OCH_2CH_3$.

$R_6$ is H, F, $CH_3$ or $=CH_2$. When $R_6$ is $=CH_2$, there is no 5-6 double bond in formula (A) or in formula (C).

Either (i) $R_9$ is absent, the 9,11-bond is a double bond and $R_{11}$ is H; (ii) $R_9$ is H or F, the 9,11-bond is a single bond, and $R_{11}$ is H, O=, α-OH or β-OH; or (iii) the 9,11-bond is a single bond and $R_9$ and $R_{11}$ together form 9β,11β-epoxide.

---- is a single or double bond.

$\overline{R}_{22}$ is $C_{1-5}$ alkyl, $CCl_3$, phenyl, phenyl substituted with $C_{1-4}$ alkyl or substituted with 1 to 3 $NO_2$ or $CF_3$ groups, $C_{7-12}$ aralkyl or $-N-(R_{122})_2$: $R_{122}$ is $C_{1-4}$ alkyl, phenyl or phthalimido.

M is Cl or Br.

∿ indicates that the attached atom or group can be in either the α or β configuration.

The following Examples illustrate the invention.

Example 1 17α-Ethynyl-3,17β-dihydroxyandrosta-3,5-diene 3-methyl ether

THF is cooled to -40°. Acetylene is bubbled through for 65 minutes keeping the temperature at -40°. 3-Hydroxyandrosta-3,5-dien-17-one 3-methyl ether (1.00 g) is added and the mixture kept at -40° for another 10 minutes while acetylene bubbling continues. The acetylene is stopped and the pot temperature is kept at less than -35°.

In a separate flask diisopropylamine (1.27 ml) in THF is cooled in an ice bath. n-Butyllithium (1.6 M), 5.64 ml) is added dropwise to the amine mixture producing lithium diisopropylamide.

The lithium diisopropylamide mixture is then added dropwise to the steroid-acetylene mixture while maintaining the temperature at less than -35°. The mixture is stirred until TLC indicates the reaction is complete. The reaction mixture is poured into phosphate buffer (40 ml) and left overnight at 20-25°. The layers are separated. The aqueous layer is back extracted with ethyl acetate. The organic phases are combined, dried over sodium sulfate, filtered and the filtrate concentrated under reduced pressure to give the title compound which is crystallized from methanol.

Example 2 17α-Ethynyl-17β-hydroxy-16β-methylandrosta-1,4,9(11)-trien-3-one (IIB)

THF (50 ml) and diisopropylamine (40 ml) are mixed and cooled to -20°. Slowly n-butyl lithium (1.6 M in hexane, 195 ml) is added with cooling maintaining the temperature in the range of -22 to -18°. When addition is complete the lithium diisopropylamide is maintained at -20°.

THF (360 ml) is saturated with acetylene by bubbling acetylene thru the THF at -20 to -15°. The acetylene saturated THF is then cooled to -25°.

The lithium diisopropylamide prepared above is added dropwise to the acetylene maintaining the reaction temperature less than -10°. When the lithium diisopropyl amide addition is complete, the monolithium acetylide is cooled to less than -40°.

16β-Methylandrosta-1,4,9(11)-triene-3,17-dione (US Patent 3,010,958, 20 g) is added in THF (60 ml) to the lithium acetylide at -40°. When the reaction is complete, acetone (24 ml) is added. The mixture is warmed while bubbling nitrogen thru the slurry to remove excess acetylene. Water (25 ml) is slowly added. Water (105 ml) is then added more quickly creating two phases which are separated. The aqueous phase is washed with THF (50 ml); the organic phases (pH is 9) are combined, washed with aqueous sulfuric acid and concentrated to 350 ml. Heptane (200 ml) is added, the mixture heated to 80° and held for 30 min. The mixture is cooled slowly to 30° and then to 18° for one hr. The mixture is filtered, the solid washed with heptane (20 ml) and then dried under reduced pressure with heat to give the title compound.

Example 3

Dry THF (5 l) is cooled to -20° and acetylene (0.27 kg) is dissolved in the THF at -20°. Diisopropylamine (880 ml) is added to the acetylene/THF solution. n-Butyllithium (1.6 M, 3.88 l) is added to the mixture, while maintaining the temperature at -20°, and stirred for 30 min.

16β-methylandrosta-1,4,9(11)-triene-3,17-dione may be added to the reaction mixture at or below -20°, and stirred for 20 min to give 17α-ethynyl-17β-hydroxy-16β-methylandrosta-1,4,9(11)-trien-3-one (IIB).

Example 4 17α-Ethynyl-17β-hydroxy-16β-methylandrosta-1,4,9(11)-trien-3-one (IIB).

THF (315 ml) and diisopropylamine (122 g) are mixed and cooled to -30°. n-Butyllithium (480 g) is added slowly to form lithium diisopropylamide, maintaining the temperature at -20 ± 1°.

THF (1.36 1) is cooled to -20° and acetylene (100 g) is dissolved in the THF using a nitrogen sweep (for safety reasons). The mixture is cooled to less than -25° and the LDA mixture is added slowly keeping the temperature less than -20°. The entire monolithium acetylide mixture is cooled to -45°.

16β-Methylandrosta-1,4,9(1l)-triene-3,17-dione (70 g) is dissolved in THF (260 ml) and cooled to 20-22°. The solution is added to the monolithium acetylide, keeping the pot temperature at -40°; the addition is complete in 10 min or less. When the reaction is complete (about 10 min at -40°), the reaction mixture is quenched with acetone (106 ml) in water (35 ml), keeping the temperature at less than -35°. The mixture is sparged with nitrogen. Water (480 ml) is added and the mixture warmed to 45°. The mixture is degassed under reduced pressure for 30 min monitoring the acetylene by GLC and then cooled to about 25°.

The reaction mixture is worked up by means well known to those skilled in the art, to give the title compound.

Example 5

Dry THF (250 ml) is cooled to -10° and sparged with acetylene until saturated. The acetylene addition is stopped and the solution cooled to -20°, whereupon diisopropylamine (44 ml) is added while maintaining -20°. n-Butyllithium (1.6 M, 194 ml) is then added, also maintaining -20°, and the resulting mixture is stirred at -20° for 30 min.

3-Methoxy-16-methyleneandrosta-3,5-dien-17-one (IIIAa; US-A-4416821; 50.0 g) in dry THF (200 ml) is added dropwise over a period of 30 min as the temperature is kept at about -20°. 15 min after addition, saline (500 ml) is added to quench the reaction which was worked up in the same manner as Example 2, to give 17α-ethynyl-3,17β-dihydroxy-16-methyleneandrosta-3,5-diene 3-methyl ether (IVAa).

Example 6

A cold (-10°) solution of dry THF (250 ml) is saturated with acetylene over 1½ hours. The acetylene addition is stopped and the solution cooled further to -20°. Triethylamine (44 ml) is added, followed by n-butyllithium (1.6 M, 194 ml), while maintaining -20°, and the resulting mixture is stirred at -20° for 30 min.

3-Methoxy-16-methyleneandrosta-3,5-diene-17-one is added over 30 min, maintaining about -20°. 15 min after addition, TLC shows the reaction to be complete, so the reaction mixture is dumped into saline (500 ml) and worked up in the same manner as Example 2, to give the same compound as Example 5.

In Divisional Application No. 90115678.6, certain 17α-ethynyl steroids are claimed per se. They are 17α-ethynyl-3,17β-dihydroxy-16β-methylandrosta-3,5-diene 3-methyl ether, 17α-ethynyl-3,17β-dihydroxy-16β-methylandrost-5-ene 3-THP ether, 17α-ethynyl-3,17β-dihydroxy-16β-methylandrosta-3,5,9(11)triene 3-methyl ether, 17α-ethynyl-17β-hydroxy-16β-methylandrost-4-en-3-one, 17α-ethynyl-17β-hydroxyl-16β-methylandrosta-4,9(11)-dien-3-one, and 17α-ethynyl-17β-hydroxy-16β-methylandrosta-1,4,9(11)-trien-3-one.

CHART A

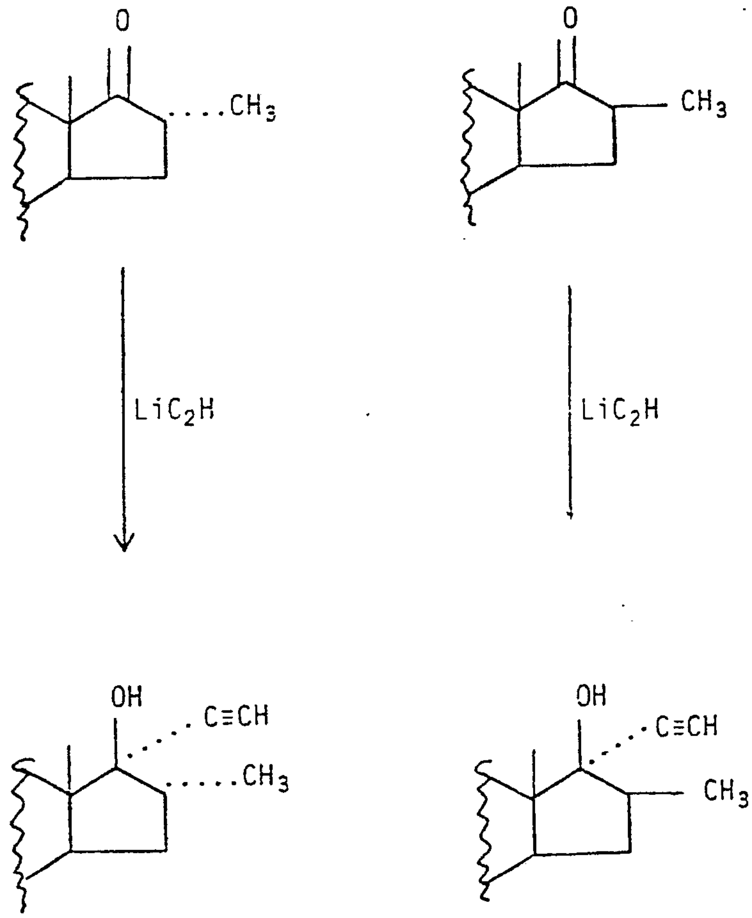


(I)

(II)

16α-Methyl

16β-Methyl

CHART A - continued

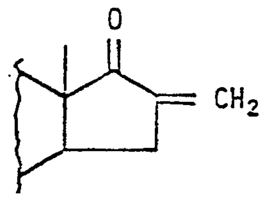

(III)

Li $C_2H$

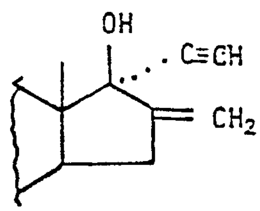

(IV)

CHART B

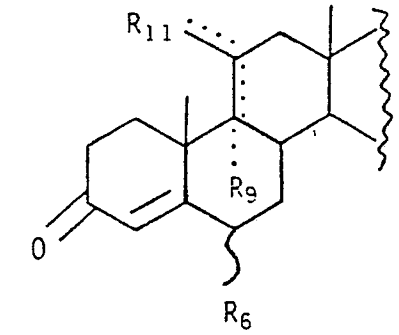

(A)

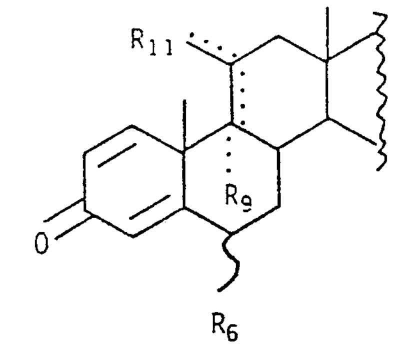

(B)

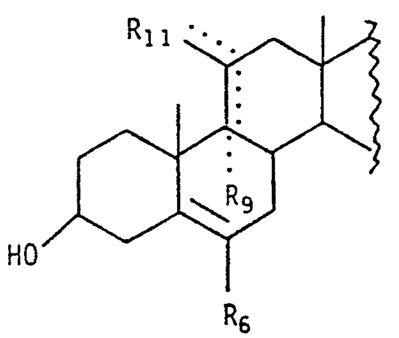

(C)

CHART C

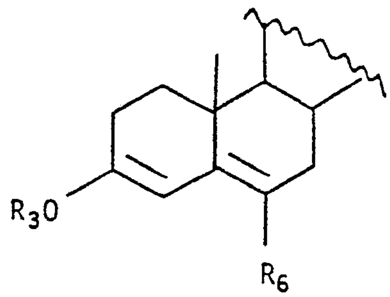

(Aa)

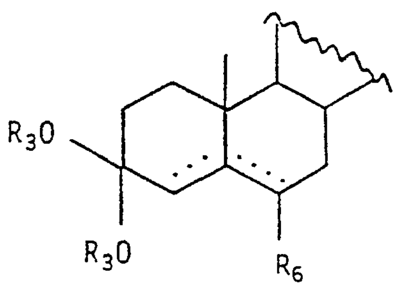

(Ab)

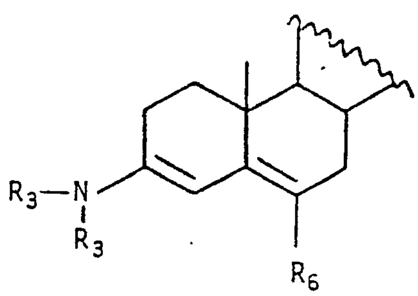

(Ac)

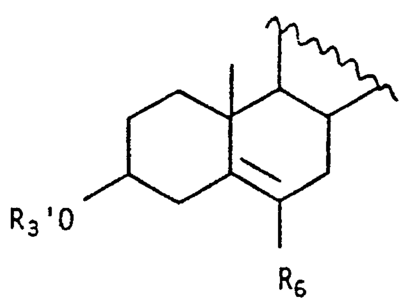

(Ca)

CHART D

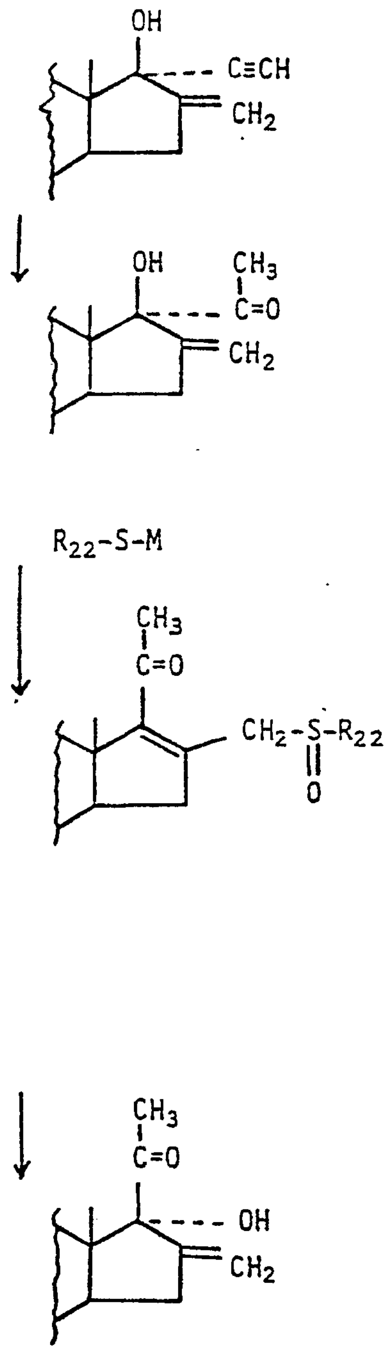


(IV)

(V)

(VII)

(VI)

(VIII)

## Claims

1. An anhydrous solution of an amine-stabilised monolithium acetylide, wherein the amine is selected from N,N-diisopropylethylamine, t-butylamine, diethylamine, dicyclohexylamine, hexamethyldisilazane and pyrrolidine.

2. A solution as claimed in claim 1, wherein the solvent is tetrahydrofuran, dioxane, diethyl ether, t-butyl methyl ether or dimethoxyethane.

3. A process for preparing a solution as claimed in claim 1 or claim 2, which comprises contacting the amine with an organolithium compound at 0 °C or below, and contacting the resultant product with a solution of acetylene in an anhydrous solvent at 0 °C or below.

4. A process for preparing an anhydrous solution of an amine-stabilised monolithium acetylide, wherein the amine is selected from N,N-diisopropylethylamine, triethylamine, diisopropylamine, t-butylamine, diethylamine, dicyclohexylamine, hexamethyldisilazane and pyrrolidine, which comprises contacting the amine with a solution of acetylene in an anhydrous solvent, and contacting the resultant product with an organolithium compound at 0 °C or below.

5. A process as claimed in claim 4, wherein the amine is diisopropylamine or triethylamine.

6. A process for preparing a 17α-ethynyl-17β-hydroxy-steroid of the formula II B

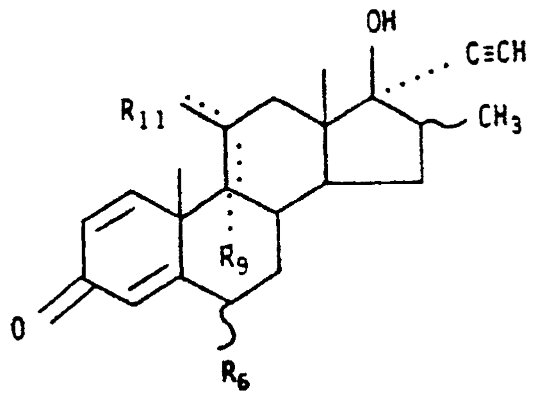

(II B)

wherein .... is a single or double bond;

$R_6$ is H, F, $CH_3$ or $=CH_2$;

either (i) $R_9$ is absent, the 9,11-bond is a double bond and $R_{11}$ is H; (ii) $R_9$ is H or F, the 9,11-bond is a single bond, and $R_{11}$ is H, O=, α-OH or β-OH; or (iii) the 9,11-bond is a single bond and $R_9$ and $R_{11}$ together form 9β,11β-epoxide; and

~ indicates α or β-configuration; which comprises the steps of:

(1) contacting a corresponding 17-keto-steroid of the formula

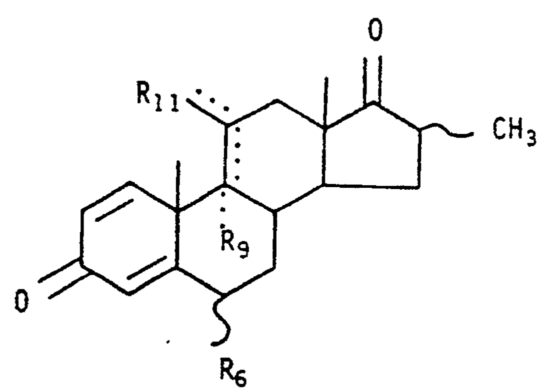

(I B)

I B with a solution of acetylene in an anhydrous solvent;

(2) contacting an amine as defined in claim 4 or claim 5 with an organolithium compound at 0 °C or below; and

(3) contacting the respective mixtures of steps (1) and (2) at 0 °C or below.

7. A process for preparing a $C_3$-protected form of a 17α-ethynyl-17β-hydroxy-steroid of the formula II A

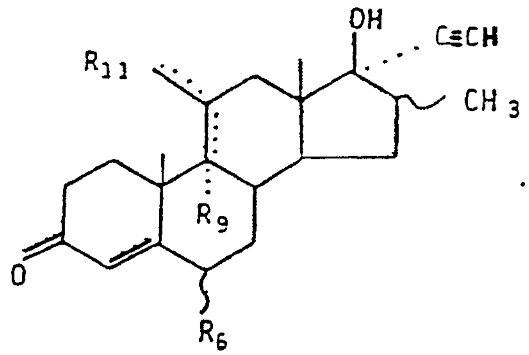

(II A)

wherein .... , $R_6$, $R_9$, and $R_{11}$ and ~ are as defined in claim 6, which comprises conducting the steps defined in claim 6 but in which the corresponding 17-keto-steroid is a $C_3$-protected form of a compound having the formula I A

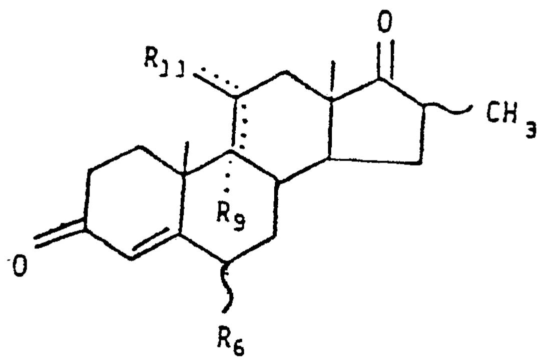

(I A)

8. A process for preparing a $C_3$-protected form of a 17α-ethynyl-17β-hydroxy-steroid of the formula II C

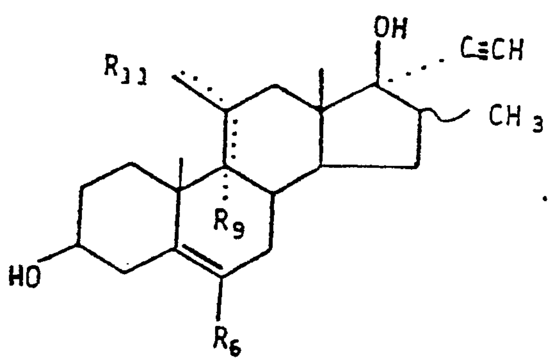

(II C)

wherein .... , $R_6$, $R_9$, and $R_{11}$ and ~ are as defined in claim 6 (except that $R_6$ is not $CH_2$), which comprises conducting the steps defined in claim 6 but in which the corresponding 17-keto-steroid is a $C_3$-protected form of a compound having the formula I C

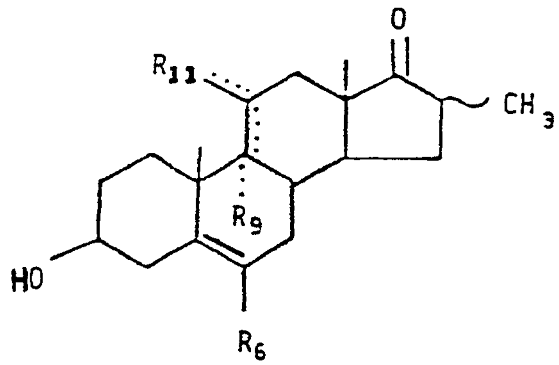

(I C)

9. A process for preparing a $C_3$-protected form of 17α-ethynyl-17β-hydroxy-steroid of the formula IV A

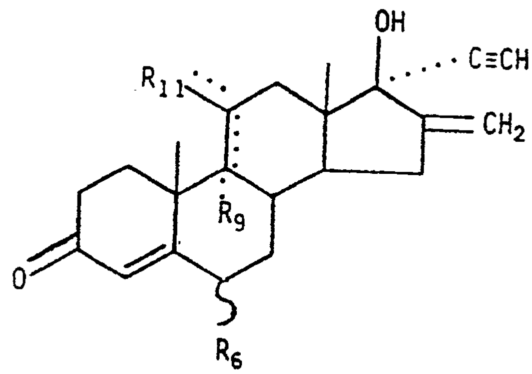

(IV A)

wherein .... , $R_6$, $R_9$, and $R_{11}$ and ~ are as defined in claim 6, which comprises conducting the steps defined in claim 6 but in which the corresponding 17-keto-steroid is a $C_3$-protected form of the compound (other than 3-methoxy-methyleneandrosta-3,5-dien-17-one) having the formula III A.

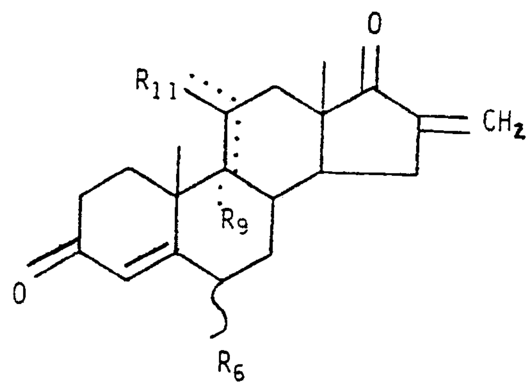

(III A)

10. A process for preparing a 17α-ethynyl-17β-hydroxy-steroid of the formula IV B

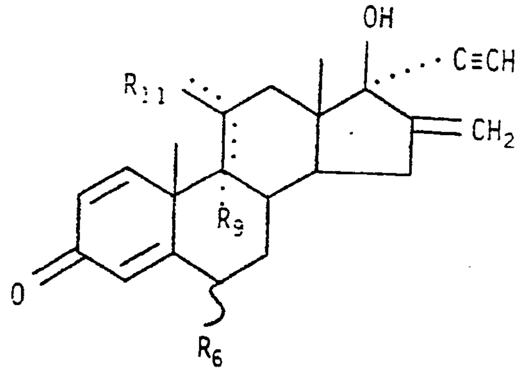

(IV B)

wherein .... , $R_6$, $R_9$, and $R_{11}$ and ∿ are as defined in claim 6, which comprises conducting the steps defined in claim 6 but in which the corresponding 17-keto-steroid is a compound having the formula III B

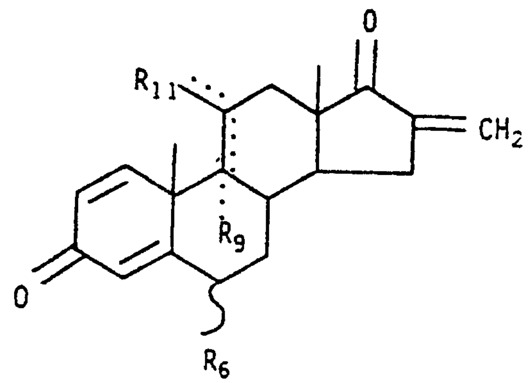

(III B)

11. A process for preparing a $C_3$-protected form of 17α-ethynyl-17β-hydroxy-steroid of the formula IV C

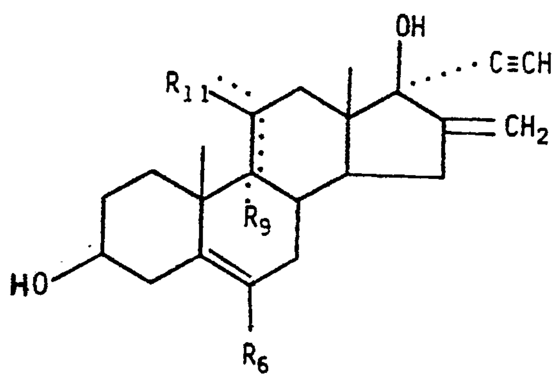

(IV C)

wherein .... , $R_6$, $R_9$, and $R_{11}$ and ∿ are as defined in claim 6 (except that $R_6$ is not $CH_2$), which comprises conducting the steps defined in claim 6 but in which the corresponding 17-keto-steroid is a $C_3$-protected form of the compound having the formula III C

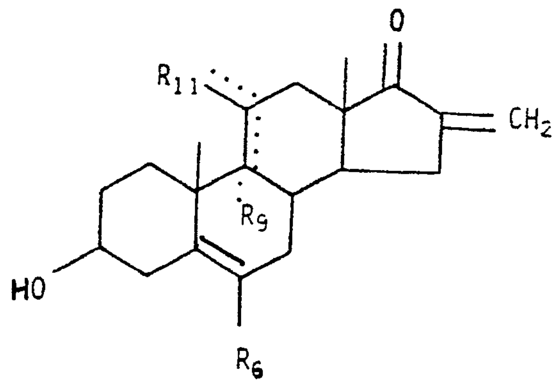

(III C)

12. A process for preparing a 17α-ethynyl-17β-hydroxy-steroid of the formula II B as defined in claim 6, which comprises the steps of

(1) contacting the corresponding 17-keto-steroid of the formula I B as defined in claim 6 with an amine-stabilised monolithium acetylide as defined in any of claims 1 to 3 in an anhydrous solvent at -10 °C or below; and

(2) while maintaining the reaction temperature at -10 °C or below, quenching the reaction with a quenching agent.

13. A process for preparing a $C_3$-protected form of a 17α-ethynyl-17β-hydroxy-steroid of the formula II A as defined in claim 7, which comprises conducting the steps defined in claim 12 but in which the corresponding 17-keto-steroid is a $C_3$-protected form of a compound having the formula I A as defined in claim 7.

14. A process for preparing a $C_3$-protected form of a 17α-ethynyl-17β-hydroxy-steroid of the formula II C as defined in claim 8, which comprises conducting the steps defined in claim 12 but in which the corresponding 17-keto-steroid is a $C_3$-protected form of a compound having the formula I C as defined in claim 8.

15. A process for preparing a $C_3$-protected form of a 17α-ethynyl-17β-hydroxy-steroid of the formula IV A as defined in claim 9, which comprises conducting the steps defined in claim 12 but in which the corresponding 17-keto-steroid is a $C_3$-protected form of a compound (other than 3-methoxy-16-methyleneandrosta-3,5-dien-17-one) having the formula III A as defined in claim 9.

16. A process for preparing a 17α-ethynyl-17β-hydroxy-steroid of the formula IV B as defined in claim 10, which comprises conducting the steps defined in claim 12 but in which the corresponding 17-keto-steroid has the formula III B as defined in claim 10.

17. A process for preparing a $C_3$-protected form of a 17α-ethynyl-17β-hydroxy-steroid of the formula IV C as defined in claim 11, which comprises conducting the steps defined in claim 12 but in which the corresponding 17-keto-steroid is a $C_3$-protected form of a compound having the formula III C as defined in claim 11.

## Revendications

1. Solution anhydre d'actéylure de monolithium stabilisé avec une amine, dans laquelle l'amine est choisie entre la N,N-diisopropyléthylamine, la tertio-butylamine, la diéthylamine, la dicyclohexylamine, l'hexaméthyldisilazane et la pyrrolidine.

2. Solution suivant la revendication 1, dans laquelle le solvant est le tétrahydrofuranne, le dioxanne, l'éther diéthylique, l'éther tertio-butylméthylique ou le diméthoxyéthane.

3. Procédé de préparation d'une solution suivant la revendication 1 ou la revendication 2, qui consiste à mettre en contact l'amine avec un composé organique de lithium à une température égale ou inférieure

à 0° C, et à mettre en contact le produit résultant avec une solution d'acétylène dans un solvant anhydre à une température égale ou inférieure à 0° C.

4. Procédé de préparation d'une solution anhydre d'acétylure de monolithium stabilisé avec une amine, dans lequel l'amine est choisie entre la N,N-diisopropyléthylamine, la triéthylamine, la diisopropylamine, la tertio-butylamine, la diéthylamine, la dicyclohexylamine, l'hexaméthyldisilazane et la pyrrolidine, qui consiste à mettre en contact l'amine avec une solution d'acétylène dans un solvant anhydre, et à mettre en contact le produit résultant avec un composé organique de lithium à une température égale ou inférieure à 0° C.

5. Procédé suivant la revendication 4, dans lequel l'amine est la diisopropylamine ou la triéthylamine.

6. Procédé de préparation d'un 17α-éthynyl-17β-hydroxy-stéroïde de formule II B

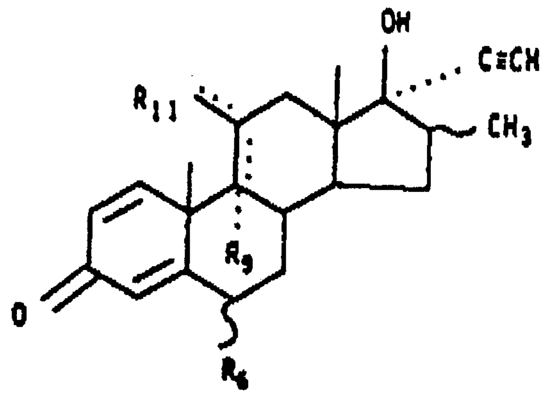

(II B)

dans laquelle .... représente une liaison simple ou une double liaison ;

$R_6$ représente H, F, un groupe $CH_3$ ou $=CH_2$ ;

soit (i) $R_9$ est absent, la liaison 9,11 est une double liaison et $R_{11}$ représente H ; soit (ii) $R_9$ représente H ou F, la liaison 9,11 est une liaison simple et $R_{11}$ représente H, O=, α-OH ou β-OH ; soit (iii) la liaison 9,11 est une liaison simple et $R_9$ et $R_{11}$ forment conjointement un groupe 9β,11β-époxyde ; et

∿ indique une configuration α ou β ; qui comprend les étapes consistant :

(1) à mettre en contact un 17-céto-stéroïde correspondant de formule I B

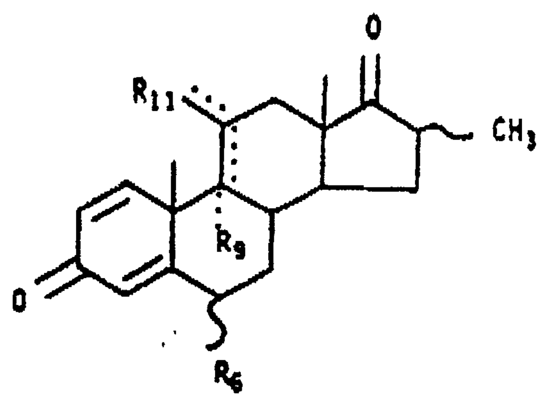

(I B)

avec une solution d'acétylène dans un solvant anhydre ;

(2) à mettre en contact une amine suivant la revendication 4 ou la revendication 5 avec un composé organique de lithium à une température égale ou inférieure à 0° ; et

(3) à mettre en contact les mélanges respectifs des étapes 1 et 2 à une température égale ou inférieure à 0° C.

7. Procédé de préparation d'une forme protégée en position $C_3$ d'un 17α-éthynyl-17β-hydroxy-stéroïde de formule II A

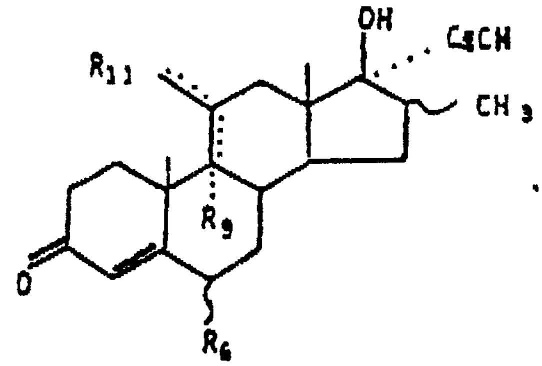

(II A)

dans laquelle .... , $R_6$, $R_9$ et $R_{11}$ et ∼ répondent aux définitions suivant la revendication 6, qui consiste à mettre en oeuvre les étapes définies dans la revendication 6, mais dans lesquelles le 17-céto-stéroïde correspondant est une forme protégée en position $C_3$ d'un composé de formule I A

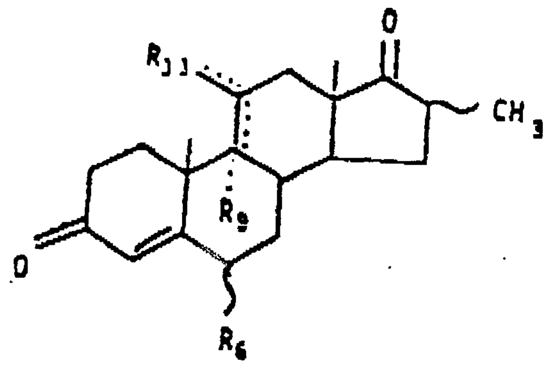

(I A)

8. Procédé de préparation d'une forme protégée en position $C_3$ d'un 17α-éthynyl-17β-hydroxy-stéroïde de formule II C

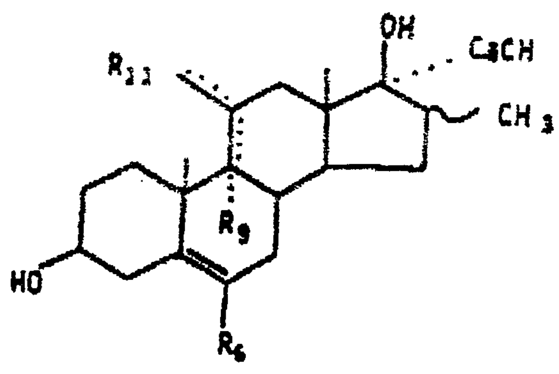

(II C)

dans laquelle .... , $R_6$, $R_9$, $R_{11}$ et ∼ répondent aux définitions selon la revendication 6 (à l'exception du fait que $R_6$ ne représente pas un groupe $CH_2$), qui consiste à mettre en oeuvre les étapes définies dans la revendication 6, mais dans lesquelles le 17-céto-stéroïde correspondant est une forme protégée en position $C_3$ d'un composé de formule I C

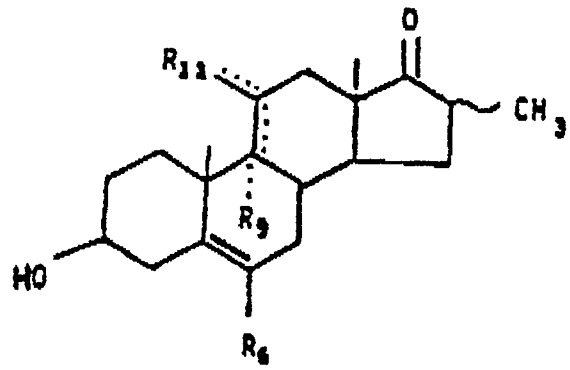

(I C)

**9.** Procédé de préparation d'une forme protégée en position $C_3$ d'un 17α-éthynyl-17β-hydroxy-stéroïde de formule IV A

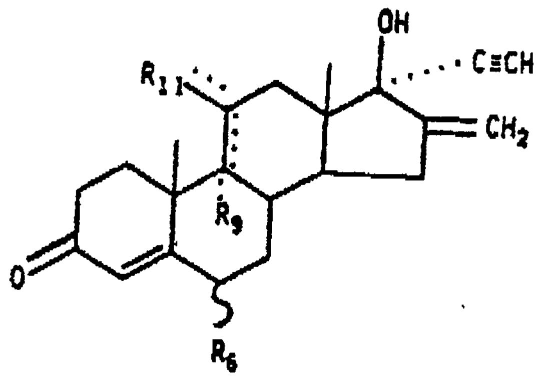

(IV A)

dans laquelle .... , $R_6$, $R_9$, $R_{11}$ et ~ répondant aux définitions suivant la revendication 6, qui consiste à mettre en oeuvre les étapes définies dans la revendication 6, mais dans lesquelles le 17-céto-stéroïde correspondant est une forme protégée en position $C_3$ du composé (autre que la 3-méthoxy-16-méthylèneandrosta-3,5-diène-17-one) de formule III A

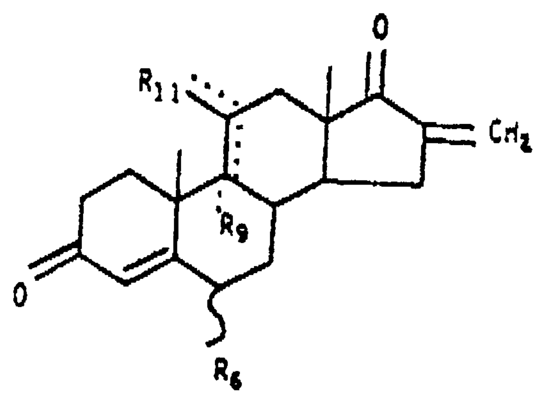

(III A)

**10.** Procédé de préparation d'un 17α-éthynyl-17β-hydroxy-stéroïde de formule IV B

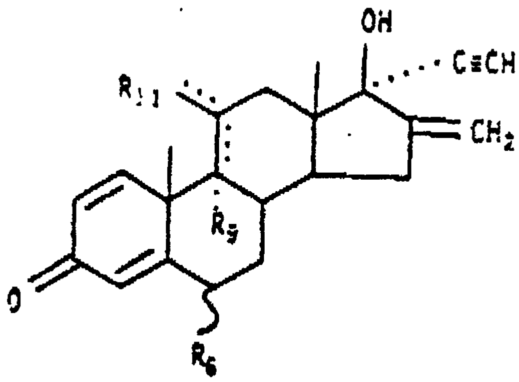

(IV B)

dans laquelle .... , $R_6$, $R_9$, $R_{11}$ et ~ répondent aux définitions suivant la revendication 6, qui consiste à mettre en oeuvre les étapes définies dans la revendication 6, mais dans lesquelles le 17-céto-stéroïde correspondant est un composé de formule III B

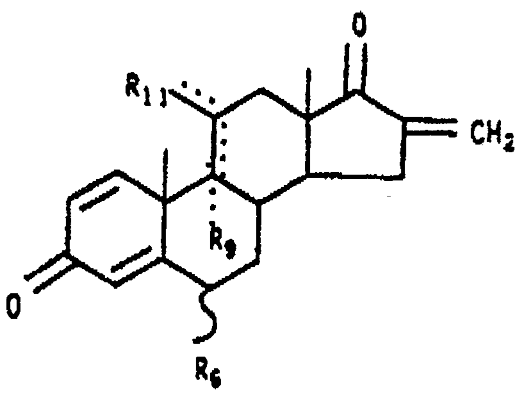

(III B)

**11.** Procédé de préparation d'une forme protégée en position $C_3$ d'un 17α-éthynyl-17β-hydroxy-stéroïde de formule IV C

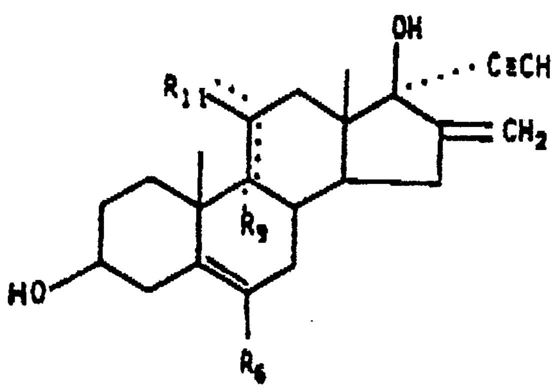

(IV C)

dans laquelle .... , $R_6$, $R_9$, $R_{11}$ et ~ répondent aux définitions suivant la revendication 6 (à l'exception du fait que $R_6$ ne représente pas un groupe $CH_2$), qui consiste à mettre en oeuvre les étapes définies dans la revendication 6, mais dans lesquelles le 17-céto-stéroïde correspondant est une forme protégée en position $C_3$ du composé de formule III C

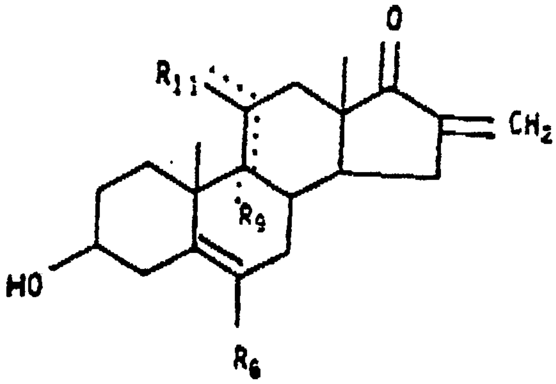

(III C)

12. Procédé de préparation d'un 17α-éthynyl-17β-hydroxy-stéroïde de formule II B suivant la revendication 6, qui comprend les étapes consistant
    (1) à mettre en contact le 17-céto-stéroïde correspondant de formule I B, suivant la revendication 6, avec un acétylure de monolithium stabilisé avec une amine suivant l'une quelconque des revendications 1 à 3 dans un solvant anhydre à une température égale ou inférieure à -10° C ;
    (2) tout en maintenant la température réactionnelle à une valeur égale ou inférieure à -10°, à désactiver la réaction avec un agent de désactivation.

13. Procédé de préparation d'une forme protégée en position $C_3$ d'un 17α-éthynyl-17β-hydroxy-stéroïde de formule II A suivant la revendication 7, qui consiste à mettre en oeuvre les étapes suivant la revendication 12, mais dans lesquelles le 17-céto-stéroïde correspondant est une forme protégée en position $C_3$ d'un composé répondant à la formule A suivant la revendication 7.

14. Procédé de préparation d'une forme protégée en position $C_3$ d'un 17α-éthynyl-17β-hydroxy-stéroïde de formule II C suivant la revendication 8, qui consiste à mettre en oeuvre les étapes suivant la revendication 12, mais dans lesquelles le 17-céto-stéroïde correspondant est une forme protégée en position $C_3$ d'un composé de formule I C suivant la revendication 8.

15. Procédé de préparation d'une forme protégée en position $C_3$ d'un 17α-éthynyl-17β-hydroxy-stéroïde de formule IV A suivant la revendication 9, qui consiste à mettre en oeuvre les étapes suivant la revendication 12, mais dans lesquelles le 17-céto-stéroïde correspondant est une forme protégée an position $C_3$ d'un composé (autre que la 3-méthoxy-16-méthylèneandrosta-3,5-diène-17-one) de formule III A suivant la revendication 9.

16. Procédé de préparation d'un 17α-éthynyl-17β-hydroxy-stéroïde de formule IV B suivant la revendication 10, qui consiste à mettre en oeuvre les étapes suivant la revendication 12, mais dans lesquelles le 17-céto-stéroïde correspondant répond à la formule III B suivant la revendication 10.

17. Procédé de préparation d'une forme protégée en position $C_3$ d'un 17α-éthynyl-17β-hydroxy-stéroïde de formule IV C suivant la revendication 11, qui consiste à mettre en oeuvre les étapes suivant la revendication 12, mais dans lesquelles le 17-céto-stéroïde correspondant est une forme protégée en position $C_3$ d'un composé répondant à la formule III C suivant la revendication 11.

## Ansprüche

1. Wasserfreie Lösung eines amin-stabilisierter Monolithiumacetylids, worin das Amin von N,N-Diisopropylethylamin, t-Butylamin, Diethylamin, Dicyclohexylamin, Hexamethyldisilazan und Pyrrolidin ausgewählt ist.

2. Lösung nach Anspruch 1, worin das Lösungsmittel Tetrahydrofuran, Dioxan, Diethylether, t-Butylmethylether oder Dimethoxyethan ist.

3. Verfahren zur Darstellung einer Lösung nach Anspruch 1 oder 2, das Verbinden des Amins mit einer

Organolithiumverbindung bei 0° C oder niedriger, und Verbinden des sich ergebenden Produktes mit einer Lösung von Acetylen in einem wasserfreien Lösungsmittel bei 0° C oder niedriger umfasst.

4. Verfahren zur Darstellung einer wasserfreien Lösung eines amin-stabilisierten Monolithiumacetylids, worin das Amin von N,N-Diisopropylethylamin, Triethylamin, Diisopropylamin, t-Butylamin, Diethylamin, Dicyclohexylamin, Hexamethyldisilazan und Pyrrolidin ausgewählt ist, welches Verbinden des Amins mit einer Lösung von Acetylen in einem wasserfreien Lösungsmittel, und Verbinden den sich ergebenden Produktes mit einer Organolithiumverbindung bei 0° C oder niedriger umfasst.

5. Verfahren nach Anspruch 4, worin das Amin Diisopropylamin oder Triethylamin ist.

6. Verfahren zur Darstellung eines 17α-Ethinyl-17β-Hydroxy-Steroids der Formel II B

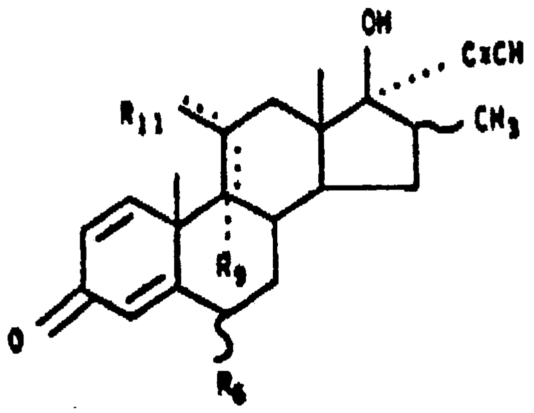

(II B)

worin .... eine Einfach- oder Doppelbindung ist;

$R_6$ H, F, $CH_3$ oder $=CH_2$ ist;

entweder (i) $R_9$ abwesend ist, die 9,11-Bindung eine Doppelbindung und $R_{11}$ H ist; (ii) $R_9$ H oder F ist, die 9,11-Bindung eine Einfachbindung ist, und $R_{11}$ H, O=, α-OH oder β-OH ist; oder (iii) die 9,11-Bindung eine Einfachbindung ist und $R_9$ und $R_{11}$ zusammen 9β,11β-Epoxid bilden; und

∿ α oder β-Konfiguration anzeigt; welches die folgenden Schritte umfasst:

(1) Verbinden eines entsprechenden 17-Keto-Steroids der Formel I B

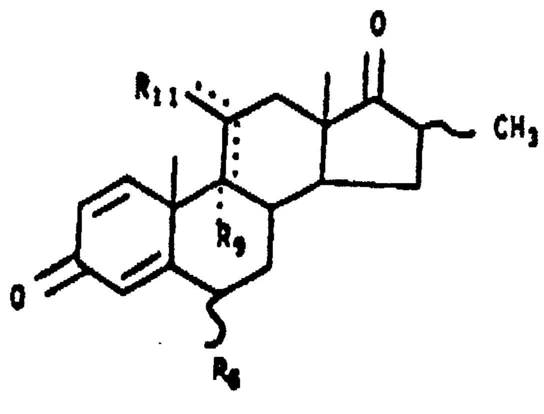

(I B)

mit einer Lösung von Acetylen in einem wasserfreien Lösungsmittel;

(2) Verbinden einen Amins wie definiert in Anspruch 4 oder Anspruch 5 mit einer Organolithiumverbindung bei 0° C oder niedriger; und (3) Verbinden der entsprechenden Mischungen dar Schritte (1) und (2) bei 0° C oder niedriger.

7. Verfahren zur Darstellung einer $C_3$-geschützten Form eines 17α-Ethinyl-17β-Hydroxy-Steroids der Formel II A

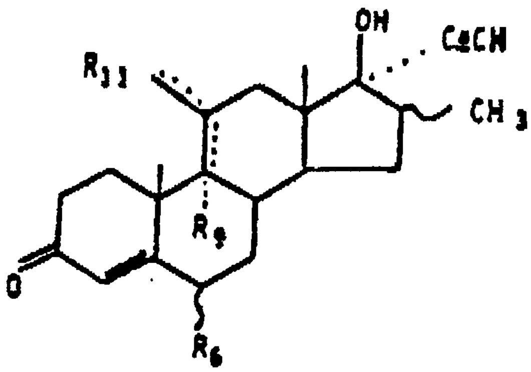

(II A)

worin .... , $R_6$, $R_9$, und $R_{11}$ und ∼ wie in Anspruch 6 definiert sind, aber in welchem das entsprechende 17-Keto-Steroid eine $C_3$-geschützte Form einer Verbindung mit der Formel I A

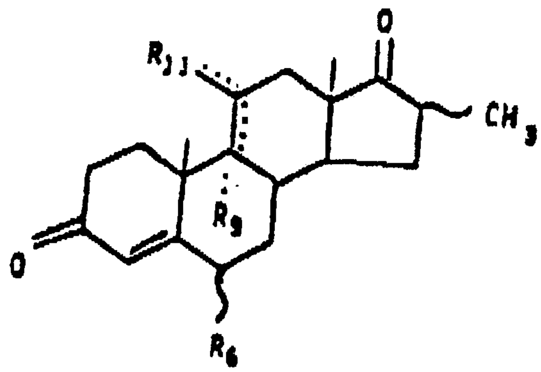

(I A)

ist.

8. Verfahren zur Darstellung einer $C_3$-geschützten Form eines 17α-Ethinyl-17β-Hydroxy-Steroids der Formel II C

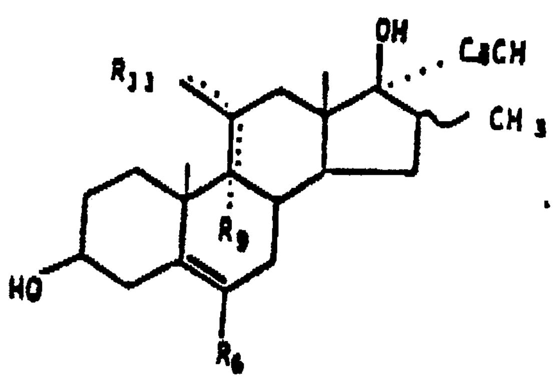

(II C)

worin .... , $R_6$, $R_9$, und $R_{11}$ und wie in Anspruch 6 definiert sind (ausser dass $R_6$ nicht $CH_2$ ist), welches Ausführen der Schritte definiert in Anspruch 6 umfasst, aber in welchem das entsprechende 17-Keto-Steroid eine $C_3$-geschützte Form einer Verbindung mit der Formel I C

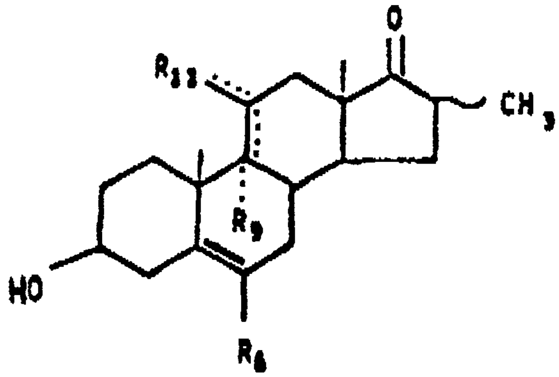

(I C)

ist.

9. Verfahren zur Darstellung einer $C_3$-geschützten Form des 17α-Ethinyl-17β-Hydroxy-Steroids der Formel IV A

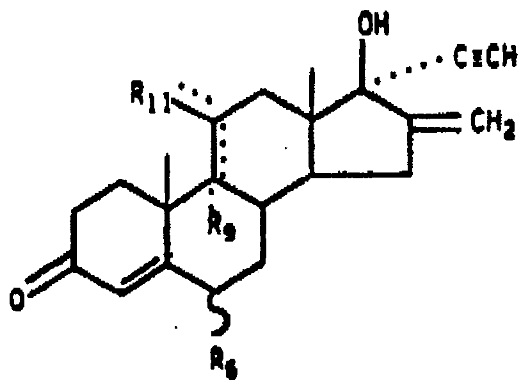

(IV A)

worin .... , $R_6$, $R_9$, und $R_{11}$ und ∿ wie in Anspruch 6 definiert sind, welches Ausführen der Schritte definiert in Anspruch 6 umfasst, aber in welchem das entsprechende 17-Keto-Steroid eine $C_3$-geschützte Form der Verbindung anders als 3-Methoxy-16-Methylenandrosta-3,5-Dien-17-On mit der Formel III A

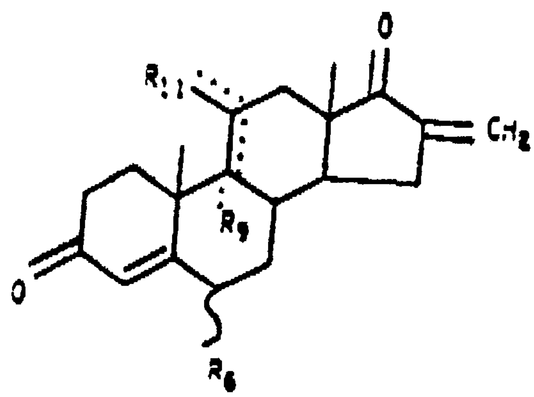

(III A)

ist.

10. Verfahren zur Darstellung eines 17α-Ethinyl-17β-Hydroxy-Steroids der Formel IV B

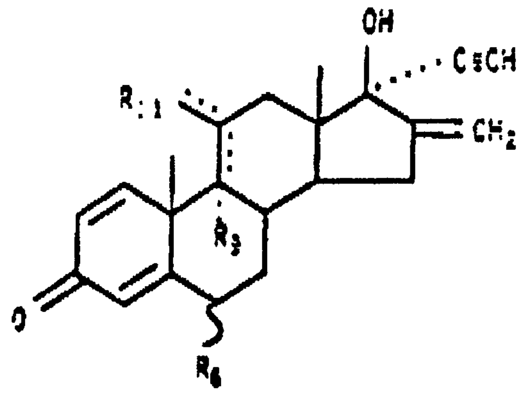

(IV B)

worin .... , $R_6$, $R_9$, und $R_{11}$ und ∿ wie in Anspruch 6 definiert sind, welches Ausführen der Schritte definiert in Anspruch 6 umfasst, aber in welchem das entsprechende 17-Keto-Steroid eine Verbindung mit der Formel III B

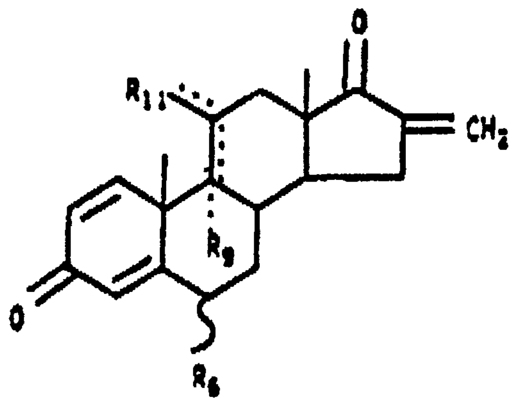

(III B)

ist.

**11.** Verfahren zur Darstellung einer $C_3$-geschützten Form des 17α-Ethinyl-17β-Hydroxy-Steroids der Formel IV C

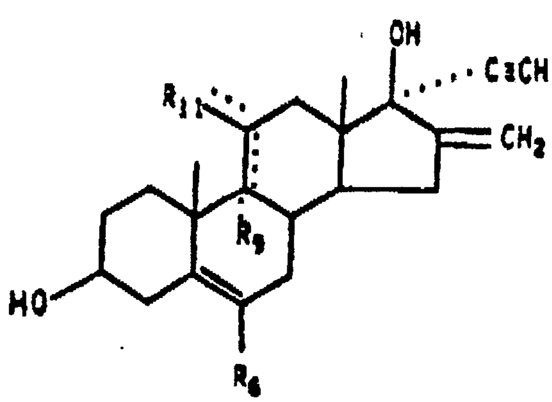

(IV C)

worin .... , $R_6$, $R_9$, und $R_{11}$ und ∿ wie in Anspruch 6 definiert sind (ausser dass $R_6$ nicht $CH_2$ ist), welches Ausführen der Schritte definiert in Anspruch 6 umfasst, aber in welchem das entsprechende 17-Keto-Steroid eine $C_3$-geschützte Form der Verbindung mit der Formel III C

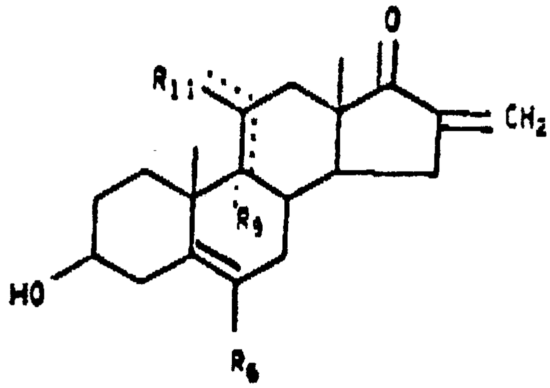

(III C)

12. Verfahren zur Darstellung eines 17α-Ethinyl-17β-Hydroxy-Steroids der Formel II B wie definiert in Anspruch 6, welches die folgenden Schritte umfasst:
(1) Verbinden des entsprechenden 17-Keto-Steroids der Formel I B wie definiert in Anspruch 6 mit einem aminstabilisierten Monolithiumacetylid wie definiert in einem der Ansprüche 1 bis 3 in einem wasserfreien Lösungsmittel bei -10° C oder niedriger; und
(2) Löschen dar Reaktion mit einem Löschmittel, während die Reaktionstemperatur bei -10° C oder niedriger beibehalten wird.

13. Verfahren zur Darstellung einer $C_3$-geschützten Form eines 17α-Ethinyl-17β-Hydroxy-Steroids der Formel II A wie definiert in Anspruch 7, welches Ausführen der Schritte wie definiert in Anspruch 12 umfasst, aber in welchem das entsprechende 17-Keto-Steroid eine $C_3$-geschützte Form einer Verbindung mit der wie in Anspruch 7 definierten Formel I A ist.

14. Verfahren zur Darstellung einer $C_3$-geschützten Form eines 17α-Ethinyl-17β-Hydroxy-Steroids der Formel II C wie definiert in Anspruch 8, welches Ausführen der Schritte wie definiert in Anspruch 12 umfasst, aber in welchem das entsprechende 17-Keto-Steroid eine $C_3$-geschützte Form einer Verbindung mit der wie in Anspruch 8 definierten Formel I C ist.

15. Verfahren zur Darstellung einer $C_3$-geschützten Form eines 17α-Ethinyl-17β-Hydroxy-Steroids der Formel IV A wie definiert in Anspruch 9, welches Ausführen der Schritte wie definiert in Anspruch 12 umfasst, aber in welchem das entsprechende 17-Keto-Steroid eine $C_3$-geschützte Form einer Verbindung anders als 3-Methoxy-16-Methylenandostra-3,5-Dien-17-On mit der wie in Anspruch 9 definierten Formel III A ist.

16. Verfahren zur Darstellung einer $C_3$-geschützten Form eines 17α-Ethinyl-17β-Hydroxy-Steroids der Formel IV B wie definiert in Anspruch 10, welches Ausführen der Schritte wie definiert in Anspruch 12 umfasst, aber in welchem das entsprechende 17-Keto-Steroid die wie in Anspruch 10 definierte Formel III B hat.

17. Verfahren zur Darstellung einer $C_3$-geschützten Form eines 17α-Ethinyl-17β-Hydroxy-Steroids der Formel IV C wie definiert in Anspruch 11, welches Ausführen der Schritte wie definiert in Anspruch 12 umfasst, aber in welchem das entsprechende 17-Keto-Steroid eine $C_3$-geschützte Form einer Verbindung mit der wie in Anspruch 11 definierten Formel III C ist.